(19) Europäisches Patentamt
European Patent Office
Office européen des brevets

(11) EP 0 555 155 B1

(12) FASCICULE DE BREVET EUROPEEN

(45) Date de publication et mention de la délivrance du brevet: **24.07.1996 Bulletin 1996/30**

(51) Int. Cl.6: **A61K 7/48**, A61K 7/06, A61K 7/08

(21) Numéro de dépôt: **93400291.6**

(22) Date de dépôt: **05.02.1993**

(54) **Composition cosmétique contenant au moins un agent tensioactif de type alkylpolyglycoside et/ou polyglycérolé et au moins un uréthannepolyéther**

Alkylpolyglycosid- und/oder Polyglycerol und Urethanpolyethertenside enthaltende kosmetische Zubereitungen

Cosmetic composition containing a surfactant such as an alkylpolyglycoside and/or polyglycerol and an urethanpolyether

(84) Etats contractants désignés:
**AT BE CH DE DK ES FR GB GR IE IT LI NL PT SE**

(30) Priorité: **07.02.1992 FR 9201419**

(43) Date de publication de la demande: **11.08.1993 Bulletin 1993/32**

(73) Titulaire: **L'OREAL**
**F-75008 Paris (FR)**

(72) Inventeurs:
- **Cauwet, Danièle**
  **F-75011 Paris (FR)**
- **Dubief, Claude**
  **F-78150 Le Chesnay (FR)**

(74) Mandataire: **Casalonga, Axel et al**
**BUREAU D.A. CASALONGA - JOSSE**
**8, avenue Percier**
**75008 Paris (FR)**

(56) Documents cités:
**EP-A- 0 260 430**
**FR-A- 1 477 048**
**FR-A- 2 328 763**
**GB-A- 2 128 627**
**US-A- 4 155 892**

EP 0 555 155 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

L'invention est relative à des compositions cosmétiques contenant au moins un agent tensio-actif non ionique de type alkylpolyglycoside et/ou polyglycérolé et au moins un uréthanne-polyéther.

Le brevet US 4 155 892 décrit essentiellement l'utilisation de certains uréthannes polyéthers pour épaissir des compositions de peinture à base de latex.

Les uréthannes-polyéthers sont en outre connus en tant qu'épaississants pour des compositions contenant des agents tensioactifs. La demande européenne EP 260 430 décrit des uréthannes-polyéthers susceptibles d'être utilisés en cosmétique pour épaissir les alcools gras oxyéthylénés contenant 8,5 à 15 moles d'oxyde d'éthylène, et le monoester de sorbitan oxyéthyléné à 20 moles d'oxyde d'éthylène.

Les agents tensioactifs de la famille des alkylpolyglycosides ou polyglycérolés ont déjà été préconisés dans des compositions lavantes pour les cheveux ou la peau. Ce sont des détergents doux, bien tolérés et biodégradables.

Les cheveux, agressés par les agents atmosphériques tels que la lumière ou les traitements chimiques, et lavés avec des bases lavantes classiques sont difficilement démêlables et cet inconvénient se trouve encore accentué dans le cas de cheveux fins. Les compositions contenant des uréthannes-polyéthers et des agents tensioactifs non ioniques décrites dans la demande européenne EP 260 430, ne confèrent pas des propriétés démêlantes.

La demanderesse vient de découvrir, de manière surprenante, que l'association, dans des compositions lavantes et/ou traitantes pour matières kératiniques, d'uréthannes-polyéthers à des agents tensioactifs non ioniques particuliers de type alkylpolyglycoside et/ou polyglycérolé conférait à ces compositions des propriétés démêlantes considérablement améliorées. Par ailleurs l'association conforme à l'invention permet d'obtenir une mousse très douce par rapport à la mousse généralement rêche résultant de l'utilisation d'agents non ioniques.

En outre, la demanderesse a constaté que les compositions contenant une telle association avaient de bonnes propriétés cosmétiques telles que de la douceur, un toucher agréable.

La présente invention a donc pour objet des compositions cosmétiques contenant au moins un agent tensioactif non ionique de type alkylpolyglycoside et/ou polyglycérolé et au moins un uréthanne polyéther.

Un autre objet de l'invention est constitué par l'utilisation de ces compositions pour le traitement et/ou le lavage des matières kératiniques telles que les cheveux, ou la peau, les cheveux étant particulièrement préférés.

Un autre objet de l'invention consiste en un procédé de traitement cosmétique des cheveux, ou de la peau au moyen des compositions conformes à l'invention; le procédé de lavage et de traitement des cheveux étant particulièrement préféré.

D'autres objets de l'invention apparaîtront à la lecture de la description et des exemples qui suivent.

Les compositions cosmétiques selon l'invention contiennent dans un milieu cosmétiquement acceptable au moins un agent tensio-actif non ionique de type alkylpolyglycoside et/ou polyglycérolé et au moins un uréthanne-polyéther.

Parmi les uréthannes-polyéthers utilisables selon l'invention on peut citer ceux répondant à la formule (I) suivante :

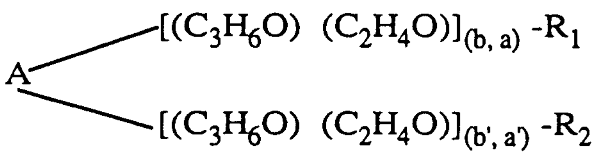

(I)

dans laquelle,

$[(C_3H_6O)(C_2H_4O)]_{(bi,\ ai)}$, avec (bi, ai) désignant (b, a) ou (b', a') signifie qu'il s'agit d'un polymère statistique d'oxyde de propylène et d'oxyde d'éthylène contenant a mole d'oxyde d'éthylène et b mole d'oxyde de propylène réparties de manière aléatoire dans la chaîne polymérique,

A désigne un radical divalent dérivé d'un diisocyanate aliphatique, cycloaliphatique ou aromatique, de préférence un radical divalent dérivé d'un polyméthylène diisocyanate, toluylène diisocyanate ou méthanediphénylène diisocyanate;

$R_1$ et $R_2$, identiques ou différents, désignent un radical alkyle ou alcényle en $C_8$-$C_{30}$, de préférence $C_{10}$-$C_{20}$, et plus particulièrement $C_{12}$-$C_{18}$;

$a_i$ et $b_i$, identiques ou différents, étant tels que la somme $a_i + b_i$ varie de 20 à 200 moles et de préférence de 60 à 120 moles;

le rapport molaire $a_i/b_i$ est compris entre $\frac{30}{70}$ et $\frac{90}{10}$, de préférence entre $\frac{50}{50}$ et $\frac{90}{10}$ et plus particulièrement entre $\frac{70}{30}$ et $\frac{85}{15}$.

Les composés de formule (I) plus particulièrement préférés sont ceux où A désigne le reste hexaméthylène diisocyanate; $R_1$ et $R_2$ désignent un radical lauryle ou un mélange de radicaux dérivés du suif; les radicaux $R_i$-$[(C_2H_4O)$-$(C_3H_6O)]_{(ai,bi)}$ ont de préférence un poids moléculaire de l'ordre de 4000, avec Ri désignant $R_1$ ou $R_2$ et $a_i$ et $b_i$ désignant a et b ou a' et b' définis ci-dessus.

Les composés de formule (I) peuvent être obtenus par réaction d'un diisocyanate avec un ou deux alcools gras polyoxyéthylénés et polyoxypropylénés, de formule

$$R_i[(OC_2H_4)\ -(OC_3H_6)]_{(ai,bi)}\ OH$$

dans laquelle $R_i$, $a_i$ et $b_i$ désigent $R_1$ ou $R_2$, a ou a' et b ou b' qui ont les significations indiquées ci-dessus, utilisé (s) en excès par rapport au diisocyanate afin que ce dernier soit totalement consommé.

Des composés de formule (I) utilisables selon l'invention sont décrits dans la demande européenne EP 260 430 et commercialisés sous la dénomination DAPRAL T210 et DAPRAL T212 par AKZO.

D'autres uréthannes polyéthers utilisables selon l'invention possèdent au moins trois groupements uréthannes. Parmi ceux-ci on peut citer ceux appartenant à l'un des trois groupes d'uréthannes polyéthers suivants :

Groupe I

Les uréthannes polyéthers répondant à la formule suivante :

$$A\text{-}B_p\text{-}E_q\text{-}(B\text{-}E)_n\text{-}B_r\text{-}E_t\text{-}A$$

dans laquelle,

n est un nombre compris entre 1 et 10, p, q, r et t, indépendamment l'un de l'autre, valent soit 0 soit 1, avec au moins l'un de q ou r valant 1, et t valant zéro lorsque r égal 0, sous réserve que lorsque q vaut 1, alors
soit p, r et t valent 0,
soit p vaut 0 et r et t valent 1,
soit t vaut 0 et r et p valent 1, et
lorsque q vaut 0, alors r vaut 1 et p et t valent 0;

Groupe II

Les uréthannes polyéthers répondant à la formule suivante II

$$(H\text{-}E\text{-}OCH_2)_sL[Q_v\text{-}(D_u\text{-}E\text{-}A)_w\text{-}R_z]_m$$

dans laquelle,

m est un nombre entier variant entre 2 et 4 et s un nombre entier variant entre 0 et 2, la somme de m et s variant entre 2 et 4, w est un nombre entier variant entre 1 et 3, et chacun de u, v et z est, indépendamment l'un de l'autre, 0 ou 1; L représente X, Y ou -O-, X étant un radical hydrocarboné hydrophobe contenant au moins un atome de carbone et de préférence un à quatre atomes de carbone, et Y un radical hydrophobe trivalent choisi parmi les groupements suivants:
$-OCONH(CH_2)_6N[CONH(CH_2)_6NHCO\text{-}O\text{-}]_2$,
$CH_3C[CH_2O\text{-}OCNHC_7H_6NHCO\text{-}]_3$, et
$CH_3CH_2C[CH_2O\text{-}OCNHC_7H_6NHCO\text{-}]_3$;
Q représente le groupe $-CH_2C-$, et D le groupe $-CH_2O-$, sous réserve que,

- a) lorsque L représente X, alors u et w valent chacun 1, v et z valent 0, m vaut au moins 2 et la somme de m et s est 4;
- b) lorsque L représente Y, alors u, v et s valent chacun 0, m vaut 3, w est 2 ou 3 et z est 0 ou 1; et
- c) lorsque L représente -O-, alors v et u valent chacun 1, w varie entre 1 et 3, m vaut 2, et s et z valent chacun 0;

dans chacune des formules de ces deux groupes, A et R représentent un radical organique hydrophobe; B un groupe divalent hydrophobe de formule :

$$-\overset{O}{\overset{\|}{C}}NH\text{-}G\text{-}NH\overset{O}{\overset{\|}{C}}\text{-}O\text{-},$$

dans laquelle G est un radical divalent dérivé d'un di- ou d'un tri-isocyanate organique dont tous les groupes isocyanate ont réagi; et E représente un groupe polyéther hydrophile divalent, non ionique.

Groupe III

Les uréthannes polyéthers sont obtenus par réaction (a) d'un réactif polyfonctionnel choisi parmi les polyols organiques possédant au moins trois groupements hydroxyl, les polyisocyanates organiques possédant au moins trois groupements isocyanates, et leur mélange ;

(b) un réactif difonctionnel choisi parmi les diols organiques, les diisocyanates organiques, et leur mélange, le diol étant présent dans le mélange de la réaction lorsque le polyisocyanate est présent et le diisocyanate étant présent lorsque le polyol est présent ;

(c) un composé monofonctionnel hydroxyl ou amino en une quantité suffisante pour piéger tout groupement isocyanate n'ayant pas réagi lors de la réaction entre (a) et (b), et pour prévenir la coagulation du mélange réactionnel; et éventuellement (d) un monoisocyanate organique pour piéger les groupements hydroxyl restant après réaction entre (a) et (b); réaction dans laquelle, au moins l'un du polyol et du diol contient au moins un segment polyéther soluble dans l'eau d'un poids moléculaire d'au moins 1 500, la somme des atomes de carbone dans les réactifs contenant des groupements isocyanate, des groupements hydroxyl et des groupements amino, est d'au moins 20, et le poids moléculaire moyen des composants de la composition est environ 10 000 à 200 000.

Les composés des groupes, I, II et II utilisables selon l'invention sont plus amplement décrits dans le brevet US 4 155 892, et commercialisés sous la dénomination ACRYSOL par la société ROHM and HAAS.

Les alkylpolyglycosides utilisables conformément à l'invention répondent en particulier à la formule (II) suivante:

$$R(C_6H_{10}O_5)_x\text{-}H \qquad (II)$$

correspondant à la structure développée (III)

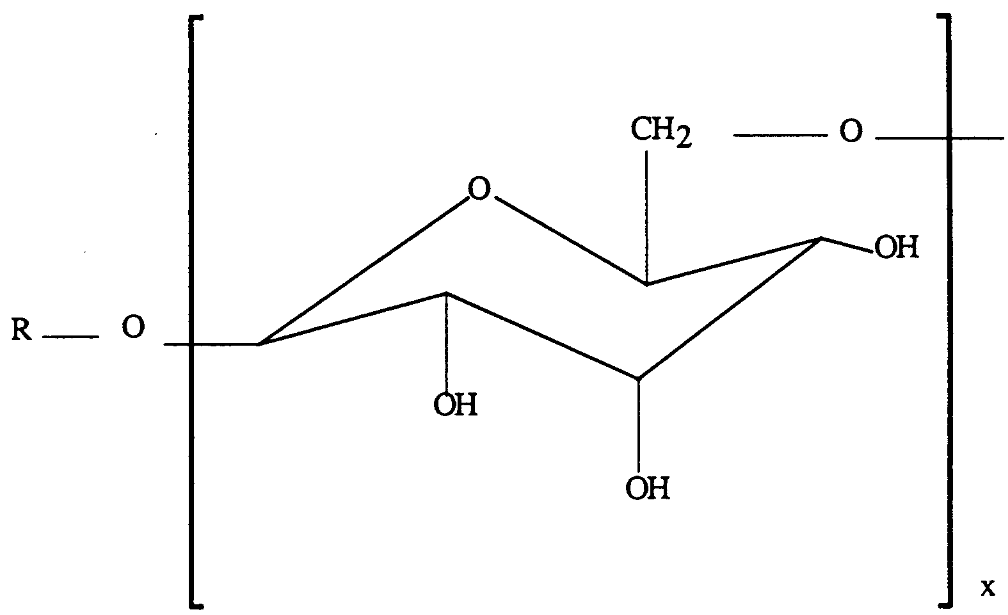

(III)

dans laquelle,

R désigne un radical ou un mélange de radicaux alkyles ou alcényles à chaîne droite ou ramifiée en $C_8$-$C_{24}$;

x est un nombre compris entre 1 et 15.

Les composés alkylpolyglycosides de formule développée (III) définie ci-dessus, utilisés conformément à l'invention, sont de préférence représentés par les produits vendus par la Société HENKEL sous la dénomination APG, tels que les produits APG 300, APG 350, APG 500, APG 550, APG 625, APG base 10-12; les produits vendus par la Société SEPPIC sous les dénominations TRITON CG 110 (ou ORAMIX CG 110) et TRITON CG 312 (ou ORAMIX NS 10); ceux vendu par la Société BASF sous la dénomination LUTENSOL GD 70.

Les agents tensio-actifs non-ioniques du type polyglycérolé utilisés conformément à la présente invention, sont choisis de préférence parmi les composés polyhydroxypropyléthers suivants :

(A) Les composés répondant à la formule (IV) :

$$RO\,[(C_3H_5(OH)]_n\!-\!H \qquad (IV)$$

dans laquelle le groupement $[C_3H_5\,(OH)]$ représente les structures suivantes; prises ensemble ou séparément

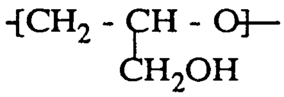 (IVb) et 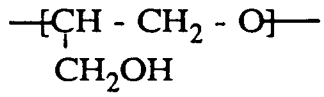 (IVc)

et R et n ont une des significations ci-après :

a) R représente un radical ou un mélange de radicaux alkyle en $C_{10}$-$C_{14}$ et n est un nombre entier ou décimal de 2 à 10 et de préférence 3 à 6.

b) R représente un reste :

$$R_2\ CONH\ CH_2 - CH_2O\ CH_2 - CH_2 - \qquad (V)$$

où $R_2$ désigne un radical ou un mélange de radicaux alkyle et/ou alcényle en $C_{11}$-$C_{17}$ et n désigne un nombre entier ou décimal de 1 à 5 et de préférence de 1,5 à 4.

c) R représente un reste :

$$R_3 - CHOH - CH_2 - \qquad (VI)$$

où $R_3$ désigne un radical aliphatique, cycloaliphatique, arylaliphatique en $C_7$-$C_{21}$ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10.

Ces tensioactifs de formule (IV) peuvent être préparés selon les procédés décrits dans les brevets FR 1 477 048, 2 328 763 et 2 091 516;

(B) Les composés préparés par condensation, en catalyse acide, de 2 à 10 et de préférence de 2, 5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol. Le procédé de préparation de ces composés est décrit dans le brevet FR-A-2 169 787;

(C) Les composés polyhydroxypropyléthers préparés par polyaddition de monochlorhydrine du glycérol sur un composé organique polyhydroxylé en présence d'une base forte, avec élimination au fur et à mesure de l'eau par distillation. Ces composés sont décrits dans le brevet français FR-A-2 574 786.

Parmi les tensio-actifs non ioniques de la famille des polyhydroxypropyléthers décrits dans les paragraphes (A), (B) et (C) ci-dessus, les composés préférés sont représentés par les formules:

(α)

$$C_{12}H_{25}O\text{-}(CH_2\text{-}\underset{\displaystyle CH_2OH}{CH}\text{-}O)_{4,2}\text{—}H \quad (VII)$$

$$R_1O\text{-}(CH_2\text{-}\underset{\displaystyle CH_2OH}{CH}\text{-}O)_{3,75}\text{—}H \quad (VIII)$$

où $R_1$ désigne un mélange de radicaux alkyles en $C_{10}H_{21}$ et $C_{12}$ $H_{25}$;

(β) les composés préparés par condensation en catalyse alcaline, de 3, 5 moles glycidol sur un alphadiol ayant 12 atomes de carbone, selon le procédé décrit dans le brevet FR-A-2 091 516;

(γ) les composés répondant à la formule :

$$R_2\text{-CONH-}CH_2\text{-}CH_2\text{-O-}CH_2\text{-}CH_2\text{-O-}(CH_2\text{-CHOH-}CH_2\text{-O})_{3,5}\,H \quad (IX)$$

où $R_2$ désigne un mélange de radicaux comprenant les radicaux alkyle et alcényle suivants : $C_{11}H_{23}$,$C_{13}H_{27}$, les radicaux dérivés des acides gras du coprah et le radical dérivé de l'acide oléique;

(δ) les composés préparés par condensation de 3,5 moles de glycidol sur un mélange d'alphadiols en $C_{11}$-$C_{14}$, décrits dans le brevet FR-A-2 091 516.

Le tensio-actif non ionique polyhydroxypropyléther obtenu par condensation de la monochlorhydrine du glycérol (2,5 moles) en présence de soude sur le dodécanediol-1,2, est plus particulièrement préféré.

Les uréthannes-polyéthers sont utilisés dans les compositions conformes à l'invention, dans des proportions comprises entre 0,1 et 10 % en poids par rapport au poids total de la composition et de préférence entre 0,3 et 5 % en poids.

Si les compositions selon l'invention ne sont pas utilisées pour le lavage des matières kératiniques, le ou les agents tensio-actifs non ioniques de type alkylpolyglycoside et/ou polyglycérolé sont utilisés dans de telles compositions dans des proportions comprises entre 0,5 et 10 % en poids par rapport au poids total de la composition. Ces compositions sont utilisées notamment comme compositions à rincer, appliquées après un shampooing, une coloration, une décoloration, une permanente, un défrisage.

Si les compositions selon l'invention sont des compositions lavantes, elles contiennent le ou les tensio-actifs non ioniques dans des proportions comprises entre 3 et 50 % en poids par rapport au poids total de la composition et plus particulièrement entre 5 et 30 % en poids.

Le pH des compositions conforme à l'invention est généralement compris entre 2 et 9 plus particulièrement entre 3 et 6.

Dans la mesure où le milieu cosmétiquement acceptable de composition selon l'invention est un milieu aqueux, il peut être constitué uniquement par de l'eau ou par un mélange d'eau et d'un solvant cosmétiquement acceptable, tel que des alcools inférieurs en $C_1$-$C_4$, comme l'éthanol, l'isopropanol, le n-butanol; les alkylèneglycols, comme l'éthylèneglycol, les éthers de glycols.

Les compositions selon l'invention peuvent se présenter sous forme de liquides plus ou moins épaissis, de gels, d'émulsions (laits ou crèmes), de lotions hydroalcooliques, de dispersions, ou de mousses aérosol.

Les compositions sont par exemples des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes anti solaires, des lotions, des laits ou des crèmes de bronzage artificiel, des crèmes ou des mousses de rasage, des lotions après rasage, des produits de maquillage pour les yeux, des fards et fonds de teint pour le visage, des shampooings, des produits pour le bain ou la douche, des compositions à rincer, à appliquer après un shampooing, une coloration, une décoloration, une permanente ou un défrisage.

Les compositions conformes à l'invention peuvent éventuellement contenir, en outre, divers additifs qui n'altèrent pas les propriétés des compositions, telles que des agents tensioactifs anioniques, cationiques, amphotères ou zwittérioniques, des agents tensioactifs non ioniques autres que ceux décrits précédemment, des polymères anioniques, non ioniques, cationiques ou amphotères, des protéines, des huiles hydrocarbonées telles que des huiles de synthèse comme les isoparaffines ou des huiles minérales, végétales ou animales, des huiles, cires, résines et/ou gommes de

silicone, des agents acidifiants ou alcalinisants, des agents conservateurs, des actifs, d'autres épaisissants, des agents de mise en suspension, des adoucissants, des filtres solaires, des parfums ou d'autres adjuvants couramment utilisés en cosmétique.

Les compositions conformes à l'invention sont appliqués sur la peau ou les cheveux dans une quantité efficace cosmétiquement, fonction de la nature de la composition.

Une application particulière des compositions selon l'invention est l'application en tant que composition de lavage et de traitement cosmétique des matières kératiniques, de préférence de la peau et des cheveux et plus particulièrement en tant que shampooing. Dans ce cas-là, le shampooing est appliqué sur les cheveux humides ou secs dans des quantités efficaces pour les laver, cette application étant suivie d'un rinçage.

Les exemples suivants sont destinés à illustrer l'invention sans pour autant présenter un caractère limitatif.

EXEMPLE 1

On prépare un shampooing de composition suivante :

| | |
|---|---|
| Alkyl ($C_9$-$C_{10}$-$C_{11}$/20-40-40)polyglycoside (1,4)vendu à 50% de MA par la société HENKEL sous la dénomination APG 300 | 15gMA |
| Uréthanne polyéther vendu sous la dénomination DAPRAL T210 par la société AKZO | 2,5g |
| Parfum, conservateur qs | |
| pH spontané 6,2 | |
| Eau qsp | 100 g |

EXEMPLE 2

On prépare un shampooing de composition suivante

| | |
|---|---|
| Alkyl($C_{10}$-$C_{12}$-$C_{14}$/85-10-5)polyglycoside(1,4) vendu à 55 % de MA sous la dénomination ORAMIX NS 10 par la société SEPPIC | 15 gMA |
| Uréthanne polyéther vendu sous la dénomination DAPRAL T212 par la société AKZO | 2 g |
| Parfum, conservateur qs | |
| pH spontané 6,2 | |
| Eau qsp | 100 g |

EXEMPLE 3

On prépare un shampooing de composition suivante

| | |
|---|---|
| Alkyl($C_{10}$-$C_{12}$-$C_{14}$/85-10-5)polyglycoside(1,4) vendu à 55 % de MA sous la dénomination ORAMIX NS 10 par la société SEPPIC | 10gMA |
| Uréthanne polyéther vendu sous la dénomination DAPRAL T210 par la société AKZO | 2 g |

Tensioactif de type acide éther carboxylique polyoxyéthyléné de formule :

$$R(OCH_2CH_2)_n\ OCH_2COOH$$

dans laquelle : R = chaîne alkyle en $C_{12}$-$C_{14}$
n = valeur moyenne de 4,5

| | |
|---|---|
| vendu sous la dénomination AKYPO RLM 45 par la société CHEM Y | 5gMA |
| Copolymère de chlorure de diallyl diméthyl ammonium et d'acrylamide vendu en solution aqueuse à 8 % de MA sous la dénomination MERQUAT S par la société MERCK | 0,5 gMA |
| Parfum, conservateur qs | |
| pH spontané 4,5 | |
| Eau qsp | 100 g |

EXEMPLE 4

On prépare un shampooing de composition suivante

| | |
|---|---|
| Tensioactif non ionique poly(hydroxypropyléther) préparé par condensation, en catalyse alcaline, de 3,5 moles de glycidol sur un mélange d'alphadiols ayant 10 à 14 atomes de carbone, selon le procédé décrit dans le brevet Freançais n° 2 091 516 | 15 g |
| Uréthanne polyéther vendu sous la dénomination DAPRAL T212 par la société AKZO | 2,5 g |
| Parfum, conservateur qs | |
| pH spontané 6,8 | |
| Eau qsp | 100 g |

EXEMPLE 5

On prépare un après-shampooing de composition suivante

| | |
|---|---|
| Alkyl($C_9$-$C_{10}$-$C_{11}$/20-40-40)polyglycoside (1,4)vendu à 50% de MA par la société HENKEL sous la dénomination APG 300 | 2 gMA |
| Uréthanne polyéther vendu sous la dénomination DAPRAL T210 par la société AKZO | 7 g |
| Chlorure de cétyltriméthylammonium vendu en solution aqueuse à 25 % de MA sous la dénomination DEHYQUART A par la société HENKEL | 1 gMA |
| Conservateur qs | |
| Hydroxyde de sodium qs | pH 6 |
| Eau qsp | 100 g |

Cette composition est appliquée sur les cheveux humides et propres. Après quelques minutes de pose les cheveux sont rincés et séchés.

EXEMPLE 6

On prépare un shampooing de composition suivante :

| | |
|---|---|
| Alkyl($C_9$-$C_{10}$-$C_{11}$/20-40-40)polyglycoside (1,4) vendu à 50 % de MA par la société HENKEL sous la dénomination APG 300 | 10 gMA |
| Uréthanne polyéther vendu sous la dénomination DAPRAL T212 par la société AKZO | 2,5 g |
| Laurylsulfate d'ammonium à 30 % de MA | 3 gMA |
| Polymère amphotère dérivé de chitosane décrit dans le brevet Français n° 2.137.684 | 1 g |
| Parfum, conservateur qs | |
| pH spontané 3,8 | |
| Eau qsp | 100 g |

EXEMPLE 7

On prépare un shampooing de composition suivante :

| | |
|---|---|
| Alkyl ($C_9$-$C_{10}$-$C_{11}$/20-40-40) polyglycoside (1,4) vendu à 50 % de MA par la société HENKEL sous la dénomination APG 300 | 15 g MA |
| Uréthanne polyéther, vendu en dispersion aqueuse à 20 % sous la dénomination ACRYSOL RM8 par la société ROHM and HAAS | 5 g MA |
| Parfum, conservateur qs | |
| HCl qs pH 7,3 | |
| Eau qsp | 100 g |

EXEMPLE 8

On prépare un shampooing de composition suivante :

| | |
|---|---|
| Alkyl ($C_9$-$C_{10}$-$C_{11}$/20-40-40) polyglycoside (1,4) vendu à 50 % de MA par la société HENKEL sous la dénomination APG 300 | 15 g MA |
| Uréthanne polyéther, vendu en dispersion aqueuse à 20 % sous la dénomination ACRYSOL RM2020 par la société ROHM and HAAS | 5 g MA |
| Parfum, conservateur qs | |
| HCl qs pH 6,7 | |
| Eau qsp | 100 g |

EXEMPLE 9

On prépare un shampooing de composition suivante :

| | |
|---|---|
| Tensioactif non ionique poly(hydrocypropyléther) préparé par condensation, en catalyse alcaline, de 3,5 moles de glycidol sur un mélange d'alphadiols ayant 10 à 14 atomes de carbone, selon le procédé dans le brevet Français n° 2.091.516 | 10 g |
| Uréthanne polyéther, vendu en dispersion aqueuse à 20 % sous la dénomination ACRYSOL RM8 par la société ROHM and HAAS | 5 g MA |
| Parfum, conservateur qs | |
| NaOH qs pH 7 | |
| Eau qsp | 100 g |

EXEMPLE 10

On prépare un shampooing de composition suivante :

| | |
|---|---|
| Alkyl $C_8$-$C_{10}$ (50/50)polyglycoside (2) vendu à 60 % de MA par la société SEPPIC sous la dénomination TRITON CG110 | 48 g MA |
| Uréthanne polyéther, vendu en dispersion aqueuse à 20 % sous la dénomination ACRYSOL RM2020 par la société ROHM and HAAS | 3 g MA |
| Parfum, conservateur qs | |
| NaOH qs pH 7 | |
| Eau qsp | 100 g |

## Revendications

1. Composition cosmétique caractérisée en ce qu'elle contient dans un milieu cosmétiquement acceptable au moins un agent tensio-actif non ionique de la famille des alkylpolyglycosides et/ou un agent tensio-actif non ionique polyglycérolé et au moins un uréthanne-polyéther.

2. Composition selon la revendication 1, caractérisée en ce que l'uréthanne-polyéther répond à la formule suivante :

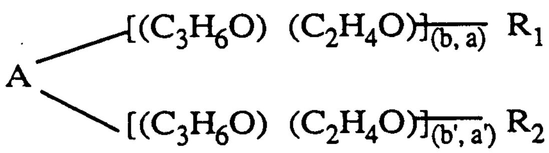

(I)

dans laquelle :

$[(C_3H_6O)(C_2H_4O)]_{(bi, ai)}$, avec ($b_i$, $a_i$) désignant (b, a) ou (b', a') signifie qu'il s'agit d'un polymère statistique d'oxyde de propylène et d'oxyde d'éthylène contenant a mole d'oxyde d'éthylène et b mole d'oxyde de propylène réparties de manière aléatoire dans la chaîne polymérique,

A désigne un radical divalent dérivé d'un diisocyanate aliphatique, cycloaliphatique ou aromatique;

$R_1$ et $R_2$, identiques ou différents, désignent un radical alkyle ou alcényle en $C_8$-$C_{30}$, de préférence $C_{10}$-$C_{20}$,

$a_i$ et $b_i$, identiques ou différents, sont tels que $a_i + b_i$, est un nombre compris entre 20 et 200, et de préférence entre 60 et 120 ;

le rapport molaire $a_i/b_i$ est compris entre $\frac{30}{70}$ et $\frac{90}{10}$, de préférence entre $\frac{50}{50}$ et $\frac{90}{10}$

**3.** Composition selon la revendication 2, caractérisée par le fait que A désigne un radical divalent dérivé d'un polyméthylène diisocyanate, toluylène diisocyanate, méthanediphénylène diisocyanate.

**4.** Composition selon l'une quelconque des revendications 1 ou 3, caractérisée en ce que A désigne le radical divalent dérivé du hexaméthylène diisocyanate et que $R_1$ et $R_2$ désignent un radical lauryle ou un mélange de radicaux dérivés du suif.

**5.** Composition selon la revendication 1, caractérisée en ce que l'uréthanne polyéther possède au moins trois groupements uréthannes.

**6.** Composition selon la revendication 5, caractérisée en ce que l'uréthanne polyéther est choisi parmi ceux représentés par les groupes suivants :

Groupe I

Les uréthannes polyéthers répondant à la formule suivante :

$$A-B_p-E_q-(B-E)_n-B_r-E_t-A$$

dans laquelle,
n est un nombre compris entre 1 et 10, p, q, r et t, indépendamment l'un de l'autre, valent soit 0 soit 1, avec au moins l'un de q ou r valant 1, et t valant zéro lorsque r égal 0, sous réserve que lorsque q vaut 1, alors
soit p, r et t valent 0,
soit p vaut 0 et r et t valent 1,
soit t vaut 0 et r et p valent 1, et
lorsque q vaut 0, alors r vaut 1 et p et t valent 0;

Groupe II

Les uréthannes polyéthers répondant à la formule suivante II

$$(H-E-OCH_2)_sL[Q_v-(D_u-E-A)_w-R_z]_m$$

dans laquelle,

m est un nombre entier variant entre 2 et 4 et s un nombre entier variant entre 0 et 2, la somme de m et s variant entre 2 et 4, w est un nombre entier variant entre 1 et 3, et chacun de u, v et z est, indépendamment l'un de l'autre, 0 ou 1; L représente X, Y ou -O-, X étant un radical hydrocarboné hydrophobe contenant au moins un atome de carbone, et de préférence un à quatre atomes de carbone et Y un radical hydrophobe trivalent choisi parmi les groupements suivants:
$-OCONH(CH_2)_6N[CONH(CH_2)_6NHCO-O-]_2$,
$CH_3C[CH_2O-OCNHC_7H_6NCO-]_3$, et
$CH_3CH_2C[CH_2O-OCNHC_7H_6NHCO-]_3$;

- Q représente le groupe $-CH_2C-$, et D le groupe $-CH_2O-$, sous réserve que,
- a) lorsque L représente X, alors u et w valent chacun 1, v et z valent 0, m vaut au moins 2 et la somme de m et s est 4;
- b) lorsque L représente Y, alors u, v et s valent chacun 0, m vaut 3, w vaut 2 ou 3 et z vaut 0 ou 1; et
- c) lorsque L représente -O-, alors v et u valent chacun 1, w varie entre 1 et 3, m vaut 2, et s et z valent chacun 0;

dans chacune des formules de ces deux groupes, A et R représentent un radical organique hydrophobe; B un groupe divalent hydrophobe de formule :

$$-\overset{O}{\overset{\|}{C}}NH-G-NH\overset{O}{\overset{\|}{C}}-O-,$$

dans laquelle G est un radical divalent dérivé d'un di- ou d'un tri-isocyanate organique dont tous les groupes isocyanates ont réagi; et E représente un groupe polyéther hydrophile divalent, non ionique.

Groupe III
Les uréthannes polyéthers sont obtenus par réaction (a) d'un réactif polyfonctionnel choisi parmi les polyols organiques possédant au moins trois groupements hydroxyl, les polyisocyanates organiques possédant au moins trois groupements isocyanate, et leur mélange ;
(b) un réactif difonctionnel choisi parmi les diols organiques, les diisocyanates organiques, et leur mélange, le diol étant présent dans le mélange de la réaction lorsque le polyisocyanate est présent et le diisocyanate étant présent lorsque le polyol est présent ;
(c) un composé monofonctionnel hydroxyl ou amino en une quantité suffisante pour piéger tout groupement isocyanate n'ayant pas réagi lors de la réaction entre (a) et (b), et pour prévenir la coagulation du mélange réactionnel; et éventuellement (d) un monoisocyanate organique pour piéger les groupements hydroxyl restant après réaction entre (a) et (b); réaction dans laquelle au moins l'un du polyol et du diol contient au moins un segment polyéther soluble dans l'eau d'un poids moléculaire d'au moins 1500, la somme des atomes de carbone dans les réactifs contenant des groupements isocyanate, des groupements hydroxyl et des groupements amino, est d'au moins 20, et le poids moléculaire moyen des composants de la compositon est environ de 10 000 à 200 000.

**7.** Composition selon l'une quelconque des revendications 1 à 6, caractérisée en ce que les agents tensioactifs non ioniques de la famille des alkylpolyglycosides répondent à la formule suivante :

$$R(C_6H_{10}O_5)_x\text{-}H \qquad (II)$$

correspondant à la structure développée suivante

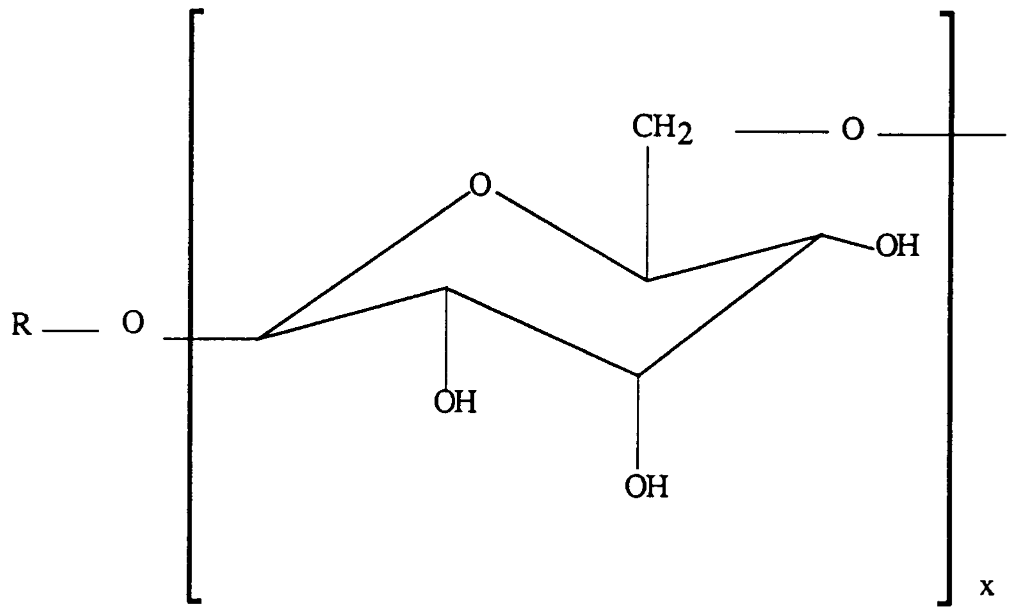

(III)

dans laquelle,
R désigne un radical ou un mélange de radicaux alkyle ou alcényle à chaîne droite ou ramifiée en $C_8$-$C_{24}$ ;
x désigne un nombre compris entre 1 et 15.

**8.** Composition selon l'une des revendications 1 à 7, caractérisée en ce que les agents tensioactifs non ioniques polyglycérolés sont choisi parmi les polyhydroxypropyléthers suivants :

(A) Les composés répondant à la formule (IV)

$$RO\ (C_3H_5\ (OH))_n\ \text{-}\ H \qquad (IV)$$

dans laquelle le groupement [$C_3$-$H_5$ (OH)] représente les structures suivantes, prises ensemble ou séparément :

$$\text{-}[CH_2CHOH\ \text{-}\ CH_2\ \text{-}O] \qquad (IVa),$$

$$-[CH_2 - \underset{CH_2OH}{CH} - O]- \quad (IVb) \qquad \text{et} \qquad -[\underset{CH_2OH}{CH} - CH_2 - O]- \quad (IVc)$$

et R et n ont une des significations ci-après :

a) R représente un radical ou un mélange de radicaux alkyle en $C_{10}$-$C_{14}$ et n est un nombre entier ou décimal de 2 à 10 et de préférence de 3 à 6.

b) R représente un reste :

$$R_2\ CONH\ CH_2 - CH_2OCH_2 - CH_2- \qquad (V)$$

où $R_2$ désigne un radical ou un mélange de radicaux alkyle et/ou alcényle en $C_{11}$-$C_{17}$ et n désigne un nombre entier ou décimal de 1 à 5 et de préférence de 1,5 à 4.

c) R représente un reste :

$$R_3 - CHOH - CH_2 - \qquad (VI)$$

où $R_3$ désigne un radical aliphatique, cycloaliphatique, arylaliphatique en $C_7$-$C_{21}$ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10.

(B) Les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol.

(C) Les composés polyhydroxypropyléthers préparés par polyaddition de monochlorhydrine du glycérol sur un composé organique polyhydroxylé en présence d'une base forte, avec élimination au fur et à mesure de l'eau par distillation.

**9.** Composition selon la revendication 8, caractérisée en ce que les agents tensioactifs non-ioniques polyglycérolés sont choisis parmi les polyhydroxypropyléthers suivants :

($\alpha$)

$$C_{12}H_{25}O\text{-}(CH_2\text{-}\underset{CH_2OH}{CH}\text{-}O)_{4,2}H \qquad (VII)$$

$$R_1O\text{-}(CH_2\text{-}\underset{CH_2OH}{CH}\text{-}O)_{3,75}H \qquad (VIII)$$

où $R_1$ désigne un mélange de radicaux alkyles en $C_{10}H_{21}$ et $C_{12}$ $H_{25}$;

($\beta$) les composés préparés par condensation en catalyse alcaline, de 3,5 moles de glycidol sur un alphadiol ayant 12 atomes de carbone.

($\gamma$) les composés répondant à la formule :

$$R_2\text{-}CONH\text{-}CH_2\text{-}CH_2\text{-}O\text{-}CH_2\text{-}CH_2\text{-}O\text{-}(CH_2\text{-}CHOH\text{-}CH_2\text{-}O)_{3,5}H \qquad (IX)$$

où $R_2$ désigne un mélange de radicaux comprenant les radicaux alkyle et alcényle suivants : $C_{11}H_{23}$, $C_{13}H_{27}$, les radicaux dérivés des acides gras du coprah et le radical dérivé de l'acide oléique;

(δ) les composés préparés par condensation de 3, 5 moles de glycidol sur un mélange d'alphadiols en $C_{11}$-$C_{14}$;

(ε) les composés obtenus par condensation de 2,5 moles de monochlorhydrine du glycérol sur le dodécanediol -1,2 en présence de soude.

**10.** Composition selon l'une quelconque des revendications 1 à 9, caractérisée en ce qu'elle renferme entre 0,1 et 10 % en poids et de préférence entre 0,3 et 5 % en poids par rapport au poids total de la composition, d'uréthannes-polyéthers.

**11.** Composition selon l'une quelconque des revendications 1 à 10, caractérisée en ce qu'elle renferme 0,5 à 10 % en poids par rapport au poids total de la composition, d'agents tensioactifs non-ioniques de la famille des alkylpolyglycosides et/ou polyglycérolés.

**12.** Composition selon l'une quelconque des revendications 1 à 10, caractérisée en ce qu'elle renferme 3 à 50 % en poids de préférence 5 à 30 % en poids par rapport au poids total de la composition, d'agents tensioactifs non-ioniques de la famille des alkylpolyglycosides et/ou polyglycérolés.

**13.** Composition selon l'une quelconque des revendications 1 à 12, caractérisée en ce que le milieu cosmétiquement acceptable est un milieu aqueux constitué uniquement par de l'eau ou par un mélange d'eau et d'un solvant cosmétiquement acceptable.

**14.** Composition selon l'une quelconque des revendications 1 à 13, caractérisée en ce qu'elle se présente sous forme de liquide plus ou moins épaissi, de gel, d'émulsion, de lotion hydroalcoolique, de dispersion, ou de mousse aérosol.

**15.** Composition selon l'une quelconque des revendications 1 à 14, caractérisée en ce qu'elle contient des agents tensioactifs anioniques cationiques, amphotères ou zwitterioniques, d'autres agents tensioactifs non ioniques, des polymères anioniques, cationiques, nonioniques ou amphotères, des protéines des huiles hydrocarbonées, des huiles, cires, résines ou gommes de silicone, des agents acidifiants ou alcalinisants, des agents conservateurs, des actifs, d'autres épaississants, des agents de mise en suspension, des adoucissants, des filtres solaires, des parfums ou d'autres adjuvants couramment utilisés en cosmétique.

**16.** Utilisation de la composition telle que définie dans l'une quelconque des revendications 1 à 15 pour le traitement cosmétique et/ou le lavage de matières kératiniques constituées par les cheveux ou la peau.

**17.** Procédé de traitement cosmétique, caractérisée en ce qu'il consiste à appliquer sur la peau ou les cheveux, une quantité cosmétiquement efficace d'une composition selon l'une quelconque des revendications 1 à 15 .

**18.** Procédé de lavage et de traitement cosmétique des cheveux caractérisé en ce qu'il consiste à appliquer sur les cheveux humides ou secs, une quantité efficace pour les laver de la composition telle que décrite dans l'une quelconque des revendications 1 à 10 et 12 à 15 et qu'on procède ensuite au rinçage à l'eau.

**Claims**

**1.** Cosmetic composition characterized in that it contains in a cosmetically acceptable medium at least one nonionic surface-active agent of the family of alkyl polyglycosides and/or one glycerolated nonionic surface-active agent and at least one polyetherurethane.

**2.** Composition according to Claim 1, characterized in that the polyetherurethane is of the following formula:

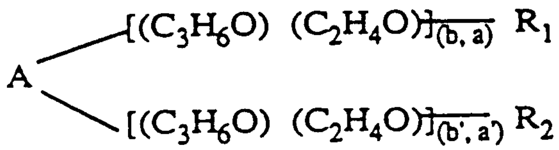

(I)

in which:
$[(C_3H_6O)\ (C_2H_4O)]_{(bi,ai)}$, with ($b_i$, $a_i$) denoting (b, a) or (b', a'), means that it is a random polymer of propylene oxide and ethylene oxide containing a mole of ethylene oxide and b mole of propylene oxide distributed in a random manner in the polymeric chain,

A denotes a divalent radical derived from an aliphatic, cycloaliphatic or aromatic diisocyanate, $R_1$ and $R_2$, which are identical or different, denote a $C_8$-$C_{30}$, preferably $C_{10}$-$C_{20}$, alkyl or alkenyl radical;

$a_i$ and $b_i$, which are identical or different, are such that the sum of between $a_i + b_i$ is a number between 20 to 200 moles, and preferably between 60 to 120 moles; the molar ratio $a_i/b_i$ is between $\frac{30}{70}$ and $\frac{90}{10}$, preferably between $\frac{50}{50}$ and $\frac{90}{10}$.

**3.** Composition according to Claim 2, characterized in that A denotes a divalent radical derived from a polymethylene diisocyanate, tolylene diisocyanate or methanediphenylene diisocyanate.

**4.** Composition according to either of Claims 1 or 3, characterized in that A denotes the divalent radical derived from hexamethylene diisocyanate and that $R_1$ and $R_2$ denote a lauryl radical or a mixture of radicals derived from tallow.

**5.** Composition according to Claim 1, characterized in that the polyetherurethane has at least three urethane groups.

**6.** Composition according to Claim 5, characterized in that the polyetherurethane is chosen from those represented by the following groups:

Group I

The polyetherurethanes of the following formula:

$$A\text{-}B_p\text{-}E_q\text{-}(B\text{-}E)_n\text{-}B_r\text{-}E_t\text{-}A$$

in which,
n is a number between 1 and 10, p, q, r and t, independently of each other, are equal to either 0 or 1, with at least one of g or r being equal to 1, and t being equal to zero when r is equal to 0, provided that when q is equal to 1, then
either p, r and t are equal to 0,
or p is equal to 0 and r and t are equal to 1,
or t is equal to 0 and r and p are equal to 1, and
when q is equal to 0, then r is equal to 1 and p and t are equal to 0;

Group II

The polyetherurethanes of the following formula II

$$(H\text{-}E\text{-}OCH_2)_sL[Q_v\text{-}(D_u\text{-}E\text{-}A)_w\text{-}R_z]_m$$

in which,

m is an integer ranging from 2 to 4 and s is an integer ranging from 0 to 2, the sum of m and s ranging from 2 to 4, w is an integer ranging from 1 to 3, and each of u, v and z is, independently of each other, 0 or 1; L represents X, Y or -O-, X being a hydrophobic hydrocarbon radical containing at least one carbon atom, and preferably one to four carbon atoms, and Y a trivalent hydrophobic radical chosen from the following groups:
$-OCONH(CH_2)_6N[CONH(CH_2)_6NHCO\text{-}O\text{-}]_2$,
$CH_3C[CH_2O\text{-}OCNHC_7H_6NHCO\text{-}]_3$
and $CH_3CH_2C[CH_2O\text{-}OCNHC_7H_6NHCO\text{-}]_3$;
Q represents the group $-CH_2C-$, and D the group $-CH_2O-$, provided that,

- a) when L represents X, then u and w are each equal to 1, v and z are equal to 0, m is equal to not less than 2 and the sum of m and s is 4;
- b) when L represents Y, then u, v and s are each equal to 0, m is equal to 3, w is 2 or 3 and z is 0 or 1; and
- c) when L represents -O-, then v and u are each equal to 1, w ranges from 1 to 3, m is equal to 2, and s and z are each equal to 0;

in each of the formulae for both of these groups, A and R represent a hydrophobic organic radical; B a hydrophobic divalent group of formula:

$$-\overset{O}{\overset{\|}{C}}NH-G-NH\overset{O}{\overset{\|}{C}}-O-,$$

in which G is a divalent radical derived from an organic di- or triisocyanate all of whose isocyanate groups have reacted; and E represents a nonionic, divalent hydrophilic polyether group.

Group III

The polyetherurethanes are obtained by reaction

(a) of a polyfunctional reagent chosen from organic polyols having at least three hydroxyl groups, organic polyisocyanates having at least three isocyanate groups, and mixtures thereof;

(b) a difunctional reagent chosen from organic diols, organic diisocyanates, and mixtures thereof, the diol being present in the reaction mixture when the polyisocyanate is present and the diisocyanate being present when the polyol is present;

(c) a hydroxyl or amino monofunctional compound in a quantity sufficient to trap any isocyanate group which did not react during the reaction between (a) and (b), and to prevent coagulation of the reaction mixture; and, optionally, (d) an organic monoisocyanate for trapping the hydroxyl groups remaining after reaction between (a) and (b); a reaction in which at least either the polyol or the diol contains at least one water-soluble polyether segment with a molecular weight of not less than 1500, the sum of the carbon atoms in the reagents containing isocyanate groups, hydroxyl groups and amino groups is not less than 20, and the average molecular weight of the components of the composition is about 10,000 to 200,000.

**7.** Composition according to any one of Claims 1 to 6, characterized in that the nonionic surface-active agents of the family of alkyl polyglycosides are of the following formula:

$$R(C_6H_{10}O_5)_x\text{-}H \qquad (II)$$

which corresponds to the following structural formula

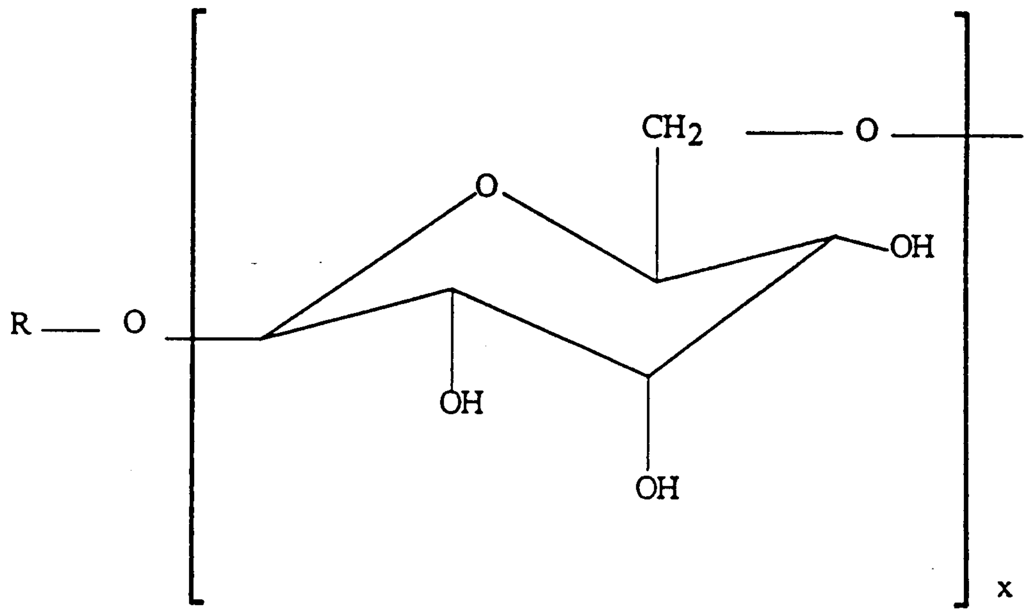

(III)

in which,
R denotes an alkyl or alkenyl radical or a mixture of alkyl or alkenyl radicals containing a linear or branched $C_8$-$C_{24}$ chain;
x is a number between 1 and 15.

8. Composition according to one of Claims 1 to 7, characterized in that the polyglycerolated nonionic surface-active agents are chosen from the following polyhydroxypropyl ethers:

(A) The compounds of the formula (IV):

$$RO(C_3H_5(OH))_nH \quad (IV)$$

in which the group [$C_3H_5(OH)$] represents the following structures taken together or separately

$$-[CH_2CHOH - CH_2 - O] \quad (IVa),$$

and

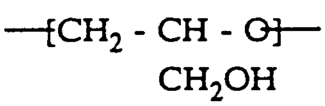 (IVb) and 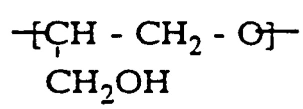 (IVc)

and R and n have one of the meanings below:

a) R represents a $C_{10}$-$C_{14}$ alkyl radical or a mixture of $C_{10}$-$C_{14}$ alkyl radicals and n is an integer or a decimal from 2 to 10, preferably 3 to 6.
b) R represents a residue:

$$R_2CONHCH_2-CH_2OCH_2-CH_2- \quad (V)$$

where $R_2$ denotes a $C_{11}$-$C_{17}$ alkyl and/or alkenyl radical or a mixture of $C_{11}$-$C_{17}$ alkyl and/or alkenyl radicals and n denotes an integer or a decimal from 1 to 5, and preferably from 1.5 to 4.
c) R represents a residue:

$$R_3\text{-CHOH-CH}_2\text{-} \quad \text{(VI)}$$

where $R_3$ denotes an aliphatic, cycloaliphatic or arylaliphatic $C_7$-$C_{21}$ radical and mixtures thereof, the aliphatic chains denoting in particular alkyl chains which may contain from 1 to 6 ether, thioether and/or hydroxymethylene groups and n denotes an integer or decimal from 1 to 10.

(B) The compounds prepared by condensation, using acid catalysis, of 2 to 10, preferably 2.5 to 6 moles of glycidol per mole of alcohol or alpha-diol containing 10 to 14 carbon atoms, at a temperature of 50 to 120°C, the glycidol being slowly added to the alcohol or alpha-diol.
(C) The polyhydroxypropyl ether compounds prepared by polyaddition of glycerol monochlorohydrin to a polyhydroxylated organic compound in the presence of a strong base, with constant removal of water by distillation.

**9.** Composition according to Claim 8, characterized in that the polyglycerolated nonionic surface-active agents are chosen from the following polyhydroxypropyl ethers:

(α)

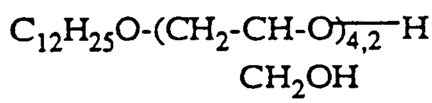

(VII)

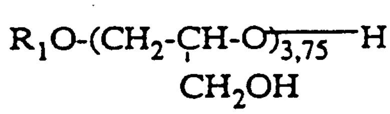

(VIII)

where $R_1$ denotes a mixture of $C_{10}H_{21}$ and $C_{12}H_{25}$ alkyl radicals;
(β) the compounds prepared by condensation, using alkaline catalysis, of 3.5 moles of glycidol with an alpha-diol having 12 carbon atoms;
(γ) the compounds of the formula:

$$R_2\text{-CONH-CH}_2\text{-CH}_2\text{-O-CH}_2\text{-CH}_2\text{-O-}(\text{CH}_2\text{-CHOH-CH}_2\text{-O})_{3.5}\text{H} \quad \text{(IX)}$$

where $R_2$ denotes a mixture of radicals comprising the following alkyl and alkenyl radicals: $C_{11}H_{23}$, $C_{13}H_{27}$, the radicals derived from fatty acids from copra and the radical derived from oleic acid;
(δ) the compounds prepared by condensation of 3.5 moles of glycidol with a mixture of $C_{11}$-$C_{14}$ alpha-diols;
(ε) the compounds obtained by condensation of 2.5 moles of glycerol monochlorohydrin with 1,2-dodecanediol in the presence of sodium hydroxide.

**10.** Composition according to any one of Claims 1 to 9, characterized in that it contains between 0.1 and 10% by weight, and preferably between 0.3 and 5% by weight relative to the total weight of the composition, of polyetherurethanes.

**11.** Composition according to any one of Claims 1 to 10, characterized in that it contains 0.5 to 10% by weight relative to the total weight of the composition, of nonionic surface-active agents of the family of alkyl polyglycosides and/or polyglycerolated agents.

**12.** Composition according to any one of Claims 1 to 10, characterized in that it contains 3 to 50% by weight, preferably 5 to 30% by weight, relative to the total weight of the composition, of nonionic surface-active agents of the family of alkyl polyglycosides and/or polyglycerolated agents.

**13.** Composition according to any one of Claims 1 to 12, characterized in that the cosmetically acceptable medium is an aqueous medium composed of water alone or of a mixture of water and a cosmetically acceptable solvent.

**14.** Composition according to any one of Claims 1 to 13, characterized in that it is provided in the form of a liquid which is more or less thickened, a gel, emulsion, dilute alcoholic lotion, dispersion or aerosol foam.

15. Composition according to any one of Claims 1 to 14, characterized in that it contains anionic, cationic, amphoteric or zwitterionic surface-active agents, other nonionic surface-active agents, anionic, cationic, nonionic or amphoteric polymers, proteins, hydrocarbon-containing oils, silicone oils, waxes, resins or gums, acidifying or alkalinizing agents, preserving agents, active ingredients, other thickeners, suspending agents, emollients, sunscreen agents, perfumes or other adjuvants commonly used in cosmetics.

16. Use of the composition as defined in any one of Claims 1 to 15 for cosmetically treating and/or washing keratinous materials consisting of the hair or the skin.

17. Process for cosmetic treatment characterized in that it consists in applying to the skin or the hair a cosmetically effective amount of the composition according to any one of Claims 1 to 15.

18. Process for the cosmetic washing and treatment of hair characterized in that it consists in applying to wet or dry hair, an effective amount, so as to wash it, of the composition as described in any one of Claims 1 to 10 and 12 to 15 and that the hair is then rinsed with water.

**Patentansprüche**

1. Kosmetische Zusammensetzung, dadurch **gekennzeichnet,** daß sie in einem kosmetischgeeigneten Milieu mindestens ein nicht-ionisches oberflächenaktives Mittel der Familie der Alkylpolyglycoside und/oder ein nicht-ionisches polyglyceriertes oberflächenaktives Mittel sowie mindestens ein Polyetherurethan enthält.

2. Zusammensetzung gemäß Anspruch 1, dadurch **gekennzeichnet,** daß das Polyetherurethan die folgende Formel aufweist:

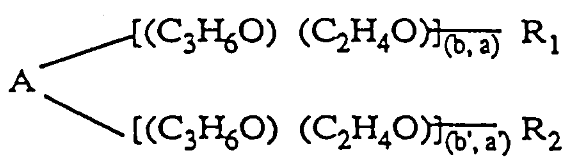

(I)

worin gilt:
$((C_3H_6O)\ (C_2H_4O))_{(bi,\ ai)}$, worin (bi, ai) (b, a) oder (b', a') bezeichnet, bedeutet, daß es sich um ein statistisches Polder aus Propylen- und Ethylenoxid handelt, enthaltend a Mol Ethylenoxid und b Mol Propylenoxid, die beliebig über die Polymerkette verteilt sind;
A bedeutet einen von einem aliphatischen, cycloaliphatischen oder aromatischen Diisocyanat abgeleiteten zweiwertigen Rest; $R_1$ und $R_2$ bedeuten, gleich oder verschieden, einen $C_{8\text{-}30}$ und vorzugsweise einen $C_{10\text{-}20}$-Alkyl oder -Alkenylrest;
$a_i$ und $b_i$ haben, gleich oder verschieden, solche Werte, daß die Summe $a_i + b_i$ 20 bis 200 und vorzugsweise 60 bis 120 Mol ausmacht;
das molare Verhältnis $a_i/b_i$ beträgt 30/70 bis 90/10, vorzugsweise 50/50 bis 90/10.

3. Zusammensetzung gemäß Anspruch 2, dadurch **gekennzeichnet,** daß A einen zweiwertigen Rest bedeutet, der von einem Polymethylen-, Toluylen- oder einem Methandiphenyldiisocyanat abgeleitet ist.

4. Zusammensetzung gemäß einem der Ansprüche 1 oder 3, dadurch **gekennzeichnet,** daß A den von Hexamethylendiisocyanat abgeleiteten zweiwertigen Rest und $R_1$ und $R_2$ einen Laurylrest oder eine Mischung aus von Talg abgeleiteten Resten bedeuten.

5. Zusammensetzung gemäß Anspruch 1, dadurch **gekennzeichnet,** daß das Polyetherurethan minidestens drei Urethan-Gruppierungen aufweist.

**6.** Zusammensetzung gemäß Anspruch 5,
dadurch **gekennzeichnet,** daß
das Polyetherurethan aus den durch die folgenden Gruppen dargestellten Verbindungen ausgewählt ist, und zwar aus:

Gruppe I:
Polyetherurethanen der folgenden Formel:

$$A\text{-}B_p\text{-}E_q\text{-}(B\text{-}E)_n\text{-}B_r\text{-}E_t\text{-}A$$

worin: gilt:
n ist eine Zahl von 1 bis 10, p, q, r und t sind, unabhängig voneinander, 0 oder 1, wobei mindestens einer der Werte für q oder r 1 beträgt, und t ist Null, wenn r gleich 0 ist, mit der Maßgabe, daß, wenn q 1 ist,
entweder p, r und t 0 sind,
oder p 0 und r und t 1 sind,
oder t 0 und r und p 1 sind,
und wenn q 0 ist, r 1 und p und t 0 sind;

Gruppe II:
Polyetherurethanen der folgenden Formel II:

$$(H\text{-}E\text{-}OCH_2)_sL(Q_v\text{-}(D_u\text{-}E\text{-}A)_w\text{-}R_z)_m$$

worin gilt:
m ist eine ganze Zahl von 2 bis 4, s ist eine ganze Zahl von 0 bis 2, die Summe von m und s beträgt 2 bis 4, w ist eine ganze Zahl von 1 bis 3, und jeder Wert für u, v und z ist, unabhängig voneinander, 0 oder 1; L stellt X, Y oder -O- dar, wobei X ein hydrophober Kohlenwasserstoffrest mit mindestens einem und vorzugsweise 1 bis 4 Kohlenstoffatomen und Y ein dreiwertiger hydrophober Rest sind, der aus den folgenden Gruppierungen ausgewählt ist:
$-OCONH(CH_2)_6N(CONH(CH_2)_6NHCO\text{-}O\text{-})_2$,
$CH_3C(CH_2O\text{-}OCNHC_7H_6NHCO\text{-})_3$ und
$CH_3CH_2C(CH_2O\text{-}OCNHC_7H_6NHCO\text{-})_3$;
Q stellt die Gruppe $-CH_2C-$ dar, und D stellt die Gruppe - $CH_2O$- dar, mit der Maßgabe, daß

- a) wenn L X darstellt, u und w jeweils 1, v und z 0, m mindestens 2 und die Summe von m und s 4 sind;
- b) wenn L Y darstellt, u, v und s jeweils 0, m 3, w 2 oder 3 und z 0 oder 1 sind; und
- c) wenn L -O- darstellt, v und u jeweils 1, w 1 bis 3, m 2 und s und z jeweils 0 sind;

in jeder der Formeln dieser beiden Gruppen stellen A und R einen organischen hydrophoben Rest dar; B stellt eine zweiwertige hydrophobe Gruppe der Formel dar:

$$-C{=}ONH\text{-}G\text{-}NHC{=}O\text{-}O\text{-},$$

worin G ein zweiwertiger, von einem organischen Di- oder Triisocyanat abgeleiteter Rest ist, wobei alle Isocyanat-Gruppen reagiert haben; und E stellt eine zweiwertige, nichtionische hydrophile Polyether-Gruppe dar;

Gruppe III:
Polyetherurethanen, die erhältlich sind durch Reaktion von

(a) einem polyfunktionellen Reaktionspartner, ausgewählt aus organischen Polyolen mit mindestens drei Hydroxylgruppen, wobei die organischen Polyisocyanate mindestens drei Isocyanatgruppen aufweisen, und aus deren Mischungen, von

(b) einem difunktionellen Reaktionspartner, ausgewählt aus organischen Diolen, organischen Diisocyanaten und deren Mischung, wobei das Diol in der Reaktionsmischung vorhanden ist, wenn das Polyisocyanat vorliegt, und wobei das Diisocyanat vorhanden ist, wenn das Polyol vorliegt, von

(c) einer monofunktionellen Hydroxyl- oder Aminoverbindung in einer ausreichenden Menge, um jede Isocyanatgruppe abreagieren zu lassen, die bei der Reaktion von (a) mit (b) nicht reagiert hat, und um eine Koagulation der Reaktionsmischung zu verhindern, sowie gegebenenfalls von

(d) einem organischen Monoisocyanat, um die nach der Reaktion von (a) und (b) verbleibenden Hydroxylgruppen abreagieren zu lassen, wobei in der Reaktion mindestens eines der Polyole und Diole mindestens ein wasserlösliches Polyether-Segment eines Molekulargewichts von mindestens 1500 enthält, die Summe der Kohlenstoffatome in den Reaktionspartnern mit Isocyanat-, Hydroxyl- und Aminogruppen mindestens 20 und das mittlere Molekulargewicht der Bestandteile der Zusammensetzung ca. 10000 bis 200000 betragen.

**7.** Zusammensetzung gemäß einem der Ansprüche 1 bis 6, dadurch **gekennzeichnet,** daß die nicht-ionischen oberflächenaktiven Mittel der Familie der Alkylpolyglycoside die folgende Formel aufweisen:

$$R(C_6H_{10}O_5)_x\text{-}H \qquad (II)$$

entsprechend der folgenden entwickelten Struktur:

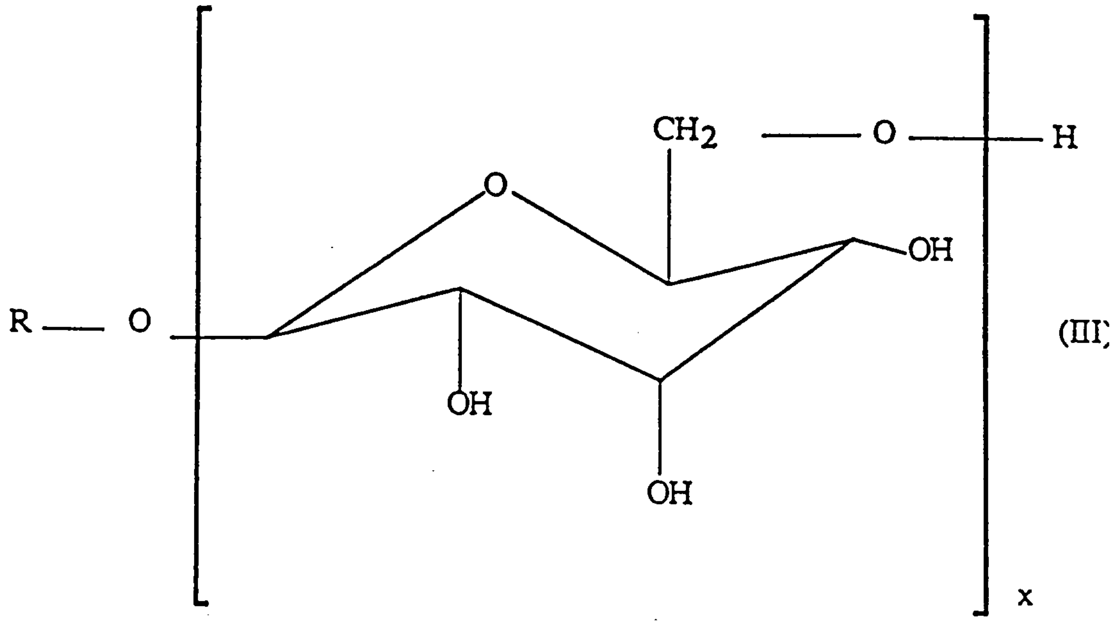

(III)

worin gilt:
R bedeutet einen Alkyl- oder Alkenylrest mit gerader oder verzweiter $C_{8\text{-}24}$-Kette oder eine Mischung dieser Reste;
x bedeutet eine Zahl von 1 bis 15.

**8.** Zusammensetzung gemäß einem der Ansprüche 1 bis 7,
dadurch **gekennzeichnet,** daß
die nicht-ionischen polyglycerierten oberflächenaktiven Mittel aus den folgenden Polyhydroxypropylethern ausgewählt sind:

(A) Verbindungen der Formel (IV):

$$RO((C_3H_5(OH))_n\text{-}H \qquad (IV)$$

worin die Gruppierung $(C_3H_5(OH))$ die folgenden Strukturen, zusammengenommen oder getrennt, darstellt:

$$\text{-}[CH_2CHOH\text{-}CH_2\text{-}O] \qquad (IVa),$$

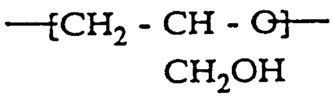 (IVb) und 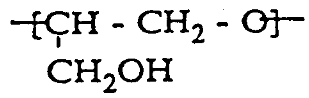 (IVc)

und worin R und n die folgenden Bedeutungen haben:

a) R stellt einen $C_{10-14}$-Alkylrest oder eine Mischung dieser Reste dar, und n ist eine ganze Zahl oder Dezimalzahl von 2 bis 10 und vorzugsweise von 3 bis 6;

b) R stellt einen Rest dar:

$$R_2CONHCH_2\text{-}CH_2OCH_2\text{-}CH_2\text{-} \qquad (V)$$

worin $R_2$ einen $C_{11-17}$-Alkyl- und/oder -Alkenylrest oder eine Mischung dieser Reste und n eine ganze Zahl oder Dezimalzahl von 1 bis 5 und vorzugsweise von 1,5 bis 4 bedeuten;

c) R stellt einen Rest dar:

$$R_3\text{-}CHOH\text{-}CH_2\text{-} \qquad (VI)$$

worin $R_3$ einen aliphatischen, cycloaliphatischen, arylaliphatischen $C_{7-21}$-Rest oder deren Mischungen bedeutet, wobei die aliphatischen Ketten insbesondere Alkylketten darstellen, die 1 bis 6 Ether-, Thioether- und/oder Hydroxymethylen-Gruppierungen aufweisen können, und worin n eine ganze Zahl oder Dezimalzahl von 1 bis 10 ist;

(B) Verbindungen, hergestellt durch unter saurer Katalyse durchgeführter Kondensation von 2 bis 10 und vorzugsweise von 2,5 bis 6 Mol Glycidol pro Mol Alkohol oder alpha-Diol mit 10 bis 14 Kohlenstoffatomen bei einer Temperatur von 50 bis 120°C, wobei das Glycidol langsam zum Alkohol oder $\alpha$-Diol gegeben wird;

(C) Polyhydroxypropylether-Verbindungen, hergestellt durch Polyaddition von Glycerylmonochlorhydrin an eine organische polyhydroxylierte Verbindung in Gegenwart einer starken Base, unter entsprechender Beseitigung des Wassers durch Destillation.

**9.** Zusammensetzung gemäß Anspruch 8, dadurch **gekennzeichnet,** daß die nicht-ionischen polyglycerierten oberflächenaktiven Mittel aus den folgenden Polyhydroxypropylethern ausgewählt sind:

($\alpha$)

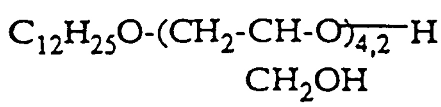 (VII)

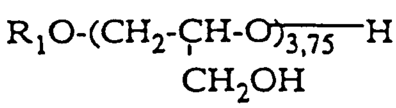 (VIII)

worin $R_1$ eine Mischung aus $C_{10}H_{21}$- und $C_{12}H_{25}$-Resten bedeutet;

($\beta$) Verbindungen, die durch Kondensation unter alkalischer Katalyse von 3,5 Mol Gycidol an ein $\alpha$-Diol mit 12 Kohlenstoffatomen hergestellt sind;

(γ) Verbindungen der Formel:

$$R_2\text{-CONH-}CH_2\text{-}CH_2\text{-O-}CH_2\text{-}CH_2\text{-O-}(CH_2\text{-CHOH-}CH_2\text{-O})_{\overline{3,5}}\text{H} \quad \text{(IX)}$$

worin $R_2$ eine Mischung von Resten bedeutet, die die folgenden Alkyl- und Alkenylreste umfassen: $C_{11}H_{23}$, $C_{13}H_{27}$, Reste, die von Fettsäuren von Kopra abgeleitet sind, sowie den von Ölsäure abgeleiteten Rest;

(δ) Verbindungen, die durch Kondensation von 3,5 Mol Glycidol an eine Mischung aus α-$C_{11-14}$-Diolen hergestellt sind;

(ε) Verbindungen, erhältlich durch Kondensation von 2,5 Mol Glycerylmonochlorhydrin an Dodecan-1,2-diol in Gegenwart von Soda.

**10.** Zusammensetzung gemäß einem der Ansprüche 1 bis 9,
dadurch **gekennzeichnet,** daß
sie 0,1 bis 10 und vorzugsweise 0,3 bis 5 Gew.% Polyetherurethane enthält, bezogen auf das Gesamtgewicht der Zusammensetzung.

**11.** Zusammensetzung gemäß einem der Ansprüche 1 bis 10,
dadurch **gekennzeichnet,** daß
sie 0,5 bis 10 Gew.% nicht-ionische oberflächenaktive Mittel der Familie der Alkylpolyglycoside und/oder polglycerierten Veribndungen enthält, bezogen auf das Gesamtgewicht der Zusammensetzung.

**12.** Zusammensetzung gemäß einem der Ansprüche 1 bis 10,
dadurch **gekennzeichnet,** daß
sie 3 bis 50 und vorzugsweise 5 bis 30 Gew.% nicht-ionische oberflächenaktive Mittel der Familie der Alkylpolyglycoside und/oder polyglycerierten Verbindungen enthält, bezogen auf das Gesamtgewicht der Zusammensetzung.

**13.** Zusammensetzung gemäß einem der Ansprüche 1 bis 12,
dadurch **gekennzeichnet,** daß
das kosmetisch geeignete Milieu ein wässriges Milieu aus Wasser allein oder aus einer Mischung aus Wasser und einem kosmetisch geeigneten Lösungsmittel ist.

**14.** Zusammensetzung gemäß einem der Ansprüche 1 bis 13,
dadurch **gekennzeichnet,** daß
sie in Form einer mehr oder weniger verdickten Flüssigkeit, eines Gels, einer Emulsion, hydroalkoholischen Lotion, Dispersion oder eines Aerosol-Schaums vorliegt.

**15.** Zusammensetzung gemäß einem der Ansprüche 1 bis 14,
dadurch **gekennzeichnet,** daß
sie anionische, kationische, amphotere oder zwitterionische oberflächenaktive Mittel, weitere nicht-ionische oberflächenaktive Mittel, anionische, kationische, nichtionische oder amphotere Polymere, Proteine, Kohlenwasserstofföle, Öle, Wachse, Harze oder Gummiprodukte aus Silicon, sauer oder alkalisch stellende Mittel, Konservierungsmittel, Wirkstoffe, weitere Verdickungsmittel, Suspendiermittel, Weichmacher, Sonnenfilterstoffe, ParfümProdukte oder weitere gewöhnlich in der Kosmetik eingesetzte Hilfsstoffe enthält.

**16.** Verwendung der in jedem der Ansprüche 1 bis 15 definierten Zusammensetzung zur kosmetischen Behandlung und/oder zum Waschen keratinischer Materien aus Haaren oder der Haut.

**17.** Verfahren zur kosmetischen Behandlung,
dadurch **gekennzeichnet,** daß
man auf die Haut oder die Haare eine kosmetisch wirksame Menge einer Zusammensetzung gemäß jedem der Ansprüche 1 bis 15 aufbringt.

**18.** Verfahren zum Waschen und kosmetischen Behandeln der Haare, dadurch **gekennzeichnet,** daß man auf die feuchten oder trockenen Haare eine zu deren Waschung wirksame Menge der in jedem der Ansprüche 1 bis 10 und 12 bis 15 beschriebenen Zusammensetzung aufbringt und anschließend mit Wasser spült.